(19) **Europäisches Patentamt**

**European Patent Office**

**Office européen des brevets**

(11) **EP 1 389 475 B1**

(12) **FASCICULE DE BREVET EUROPEEN**

(45) Date de publication et mention
de la délivrance du brevet:
**17.05.2006 Bulletin 2006/20**

(51) Int Cl.:
*A61M 1/16* (2006.01)

(21) Numéro de dépôt: **03024490.9**

(22) Date de dépôt: **07.08.1998**

(54) **Procédé pour régler la concentration du sodium dans un liquide de dialyse en vue d'une prescription**

Verfahren zur Kontrolle der Natriumkonzentration eines Dialysats nach Verordnung

Process for controlling sodium concentration in a dialysate as prescribed

(84) Etats contractants désignés:
**AT BE CH DE ES FR GB IT LI NL SE**

(30) Priorité: **21.08.1997 FR 9710671**

(43) Date de publication de la demande:
**18.02.2004 Bulletin 2004/08**

(62) Numéro(s) de document de la (des) demande(s)
initiale(s) en application de l'article 76 CBE:
**98420143.4 / 0 898 975**

(73) Titulaire: **Gambro Industries SAS**
**69330 Meyzieu (FR)**

(72) Inventeur: **Bene, Bernard**
**69540 Irigny (FR)**

(74) Mandataire: **Sutto, Luca**
**Gambro Intellectual Property Department,**
**P.O. Box 98**
**41037 Mirandola (MO) (IT)**

EP 1 389 475 B1

**Description**

La présente invention a pour objet un algorithme programmé pour usage sur un appareil de dialysie afin de mettre en oeuvre un procédé pour régler la concentration de sodium dans un liquide de dialyse en vue d'une prescription.

Les reins remplissent de multiples fonctions parmi lesquelles l'élimination d'eau, l'excrétion des catabolites (ou déchets du métabolisme, tels que l'urée, la créatinine), la régulation de la concentration des électrolytes du sang (sodium, potassium, magnésium, calcium, bicarbonates, phosphates, chlorures) et la régulation de l'équilibre acido-basique du milieu intérieur qui est obtenue notamment par l'élimination des acides faibles (phosphates, acides monosodiques) et par la production de sels d'ammonium.

Chez les personnes qui ont perdu l'usage de leurs reins, ces mécanismes excréteurs et régulateurs ne jouant plus, le milieu intérieur se charge en eau et en déchets du métabolisme, présente un excès d'électrolytes (de sodium en particulier), ainsi que, en général, une acidose, le pH du plasma sanguin se déplaçant vers 7 (le pH sanguin varie normalement dans des limites étroites comprises entre 7,35 et 7,45).

Pour pallier le dysfonctionnement des reins, on recourt classiquement à un traitement du sang par circulation extracorporelle dans un échangeur à membrane semi-perméable (hémodialyseur) où l'on fait circuler, d'un côté et de l'autre de la membrane, le sang du patient et un liquide de dialyse comprenant les principaux électrolytes du sang, dans des concentrations proches de celles du sang d'un sujet sain. Par ailleurs, on crée une différence de pression entre les deux compartiments de l'hémodialyseur délimités par la membrane semi-perméable de façon qu'une fraction de l'eau du plasma passe par ultrafiltration au travers de la membrane dans le compartiment du liquide de dialyse.

Le traitement du sang qui s'effectue dans l'hémodialyseur en ce qui concerne les déchets du métabolisme et les électrolytes résulte de deux mécanismes de transfert des molécules au travers de la membrane. D'une part, les molécules migrent du liquide où leur concentration est la plus élevée vers le liquide où leur concentration est la moins élevée. C'est le transfert par diffusion, ou dialyse. D'autre part, certains catabolites et certains électrolytes sont entraînés par l'eau plasmatique qui filtre au travers de la membrane sous l'effet de la différence de pression créée entre les deux compartiments de l'échangeur. C'est le transfert par convection.

Trois des fonctions du rein rappelées plus haut, savoir l'élimination de l'eau, l'excrétion des catabolites et la régulation de la concentration électrolytique du sang, sont donc assurées dans un dispositif classique de traitement du sang par la combinaison de la dialyse et de la filtration du sang (cette combinaison est appelée hémodialyse).

En ce qui concerne la régulation de l'équilibre acido-basique du milieu intérieur, on agit, pour pallier la déficience des reins, sur un mécanisme régulateur de l'équilibre acido-basique du milieu intérieur constitué par les systèmes tampons du sang, dont le principal comprend l'acide carbonique, comme acide faible, associé à son sel alcalin, le bicarbonate. C'est ainsi que, pour corriger l'acidose d'un patient souffrant d'insuffisance rénale, on lui administre du bicarbonate par la voie vasculaire, directement ou indirectement, au cours d'une séance d'hémodialyse.

L'administration est indirecte lorsque le bicarbonate rentre dans la composition du liquide de dialyse et passe dans le sang par diffusion. Un inconvénient de cette méthode est lié au fait que le bicarbonate précipite avec le calcium et le magnésium, qui font partie des composants classiques d'un liquide de dialyse. Pour limiter cette réaction, on ajoute un acide (acide acétique) au liquide de dialyse pour en abaisser le pH, ce qui a pour corollaire d'y augmenter la pression partielle de dioxyde de carbone et pour conséquence indésirable d'induire chez le patient un malaise résultant de l'excès de ce gaz dans son sang. En outre, compte tenu de la concentration maximale acceptable d'acide dans le liquide de dialyse, il se produit des dépôts de calcium dans les circuits de la machine de dialyse qui doivent être éliminés.

L'administration du bicarbonate est directe lorsque le liquide de dialyse est dépourvu de bicarbonate et que le patient est perfusé avec une solution de bicarbonate de sodium. Cette méthode présente l'intérêt qu'elle dispense d'avoir à mettre en présence, dans un même liquide de traitement, les substances qui précipitent en l'absence d'acide.

En revanche, elle pose le problème, du réglage de la concentration du sodium dans le liquide de dialyse pour que le milieu intérieur du patient tende vers une concentration déterminée en sodium. En effet, en perfusant du bicarbonate de sodium au patient en vue d'atteindre une concentration déterminée en bicarbonate dans le milieu intérieur du patient, on y introduit une quantité de sodium. En d'autres termes, avec les systèmes existants utilisés pour mettre en oeuvre la méthode qui vient d'être décrite, il n'est pas prévu de régler à la fois le débit de perfusion de la solution de bicarbonate de sodium et la concentration du liquide de dialyse en sodium de façon que le milieu intérieur du patient tende à la fois vers une concentration précise prédéterminée en bicarbonate et en sodium.

Le document EP 0 532 433 A1 sur lequel le préambule de la revendication 1 est basée décrit un rein artificiel comprenant :

- un échangeur ayant deux compartiments séparés par une membrane, le premier compartiment étant relié à un circuit pour circulation extracorporelle de sang, le second compartiment étant relié à un circuit de dialyse ayant une canalisation d'alimentation en liquide de dialyse ayant une canalisation d'alimentation en liquide de dialyse frais connectée à une entrée du second compartiment , et une canalisation d'alimentation en liquide de dialyse usé connectée à une sortie du second compartiment,

- des moyens pour commander des moyens de réglage du débit d'écoulement d'au moins une solution concentrée contenant une substance dans un réservoir de préparation de liquide de dialyse, la substance pouvant être du sodium ;
- des moyens de réglage de débit d'écoulement d'un liquide de perfusion dans le circuit pour circulation extracorporelle de sang.

L'unité décrit dans EP 0 532 433 compare la concentration en substances ionisées du sang du patient à la concentration souhaitée préalablement mise en mémoire et commande une pompe pour augmenter ou diminuer le débit de la solution concentrée contenant du sodium pour augmenter or diminuer la teneur en sodium du liquide de dialyse.

Le but de l'invention est de réaliser un algorithme programmé qui, une fois mis en oeuvre par une unité de commande et de calcul d'un appareil de dialyse, rend l'unité capable d'exécuter un procédé de réglage de la concentration en sodium d'un liquide de dialyse, qui permette au milieu intérieur d'un patient recevant une perfusion d'une solution de sel de sodium de tendre précisément vers une concentration souhaitée en sodium [Na$^+$]des.

Pour atteindre ce but, on prévoit, conformément à l'invention un algorithm programmé selon la revendication 1 qui, une fois en oeuvre rend l'unité de commande et de calcul capable d'éxécuter un procédé pour régler la concentration en sodium d'un liquide de dialyse à une valeur déterminée [Na$^+$]dial pour que le milieu intérieur d'un patient tende vers une concentration souhaitée en sodium [Na$^+$]$_{des}$, le sang du patient et le liquide de dialyse étant mis en circulation de part et d'autre de la membrane semi-perméable d'un dialyseur, le patient recevant par ailleurs une perfusion d'une solution contenant du sodium et une substance A à des concentrations déterminées [Na$^+$]$_{sol}$ et [A]$_{sol}$;

le procédé comprenant les étapes de:

- déterminer la concentration en sodium [Na$^+$]$_{dial}$ du liquide de dialyse en fonction de la dialysance D du dialyseur pour le sodium, de la concentration souhaitée en sodium [Na$^+$]$_{des}$ du milieu intérieur du patient, du débit de perfusion Qinf et de la concentration en sodium [Na$^+$]$_{sol}$ de la solution de perfusion ; et
- ajuster la concentration en sodium du liquide de dialyse à la concentration déterminée [Na$^+$]$_{dial}$.

De préférence, selon une caractéristique de invention l'étape de déterminer la concentration en sodium [Na$^+$]$_{dial}$ du liquide de dialyse consiste à appliquer la formule :

$$[Na^+]dial = \frac{Q_{inf}}{D} ([Na+]des - [Na+]sol) + [Na+]des$$

De préférence, selon une autre caractéristique de l'invention, le procédé comporte en outre l'étape de déterminer la dialysance D du dialyseur pour le sodium selon les étapes de:

- préparer et faire circuler successivement dans le dialyseur au moins deux liquides de dialyse ayant des conductivités différentes ;
- prendre au moins deux mesures de la conductivité d'au moins deux liquides de dialyse en amont et en aval du dialyseur;
- calculer la dialysance D à partir des valeurs de conductivité mesurées (Cd1 in, Cd1out, Cd2in, Cd2out) dans au moins deux liquides de dialyse préparés successivement.

De préférence, la dialysance D du dialyseur pour le sodium est calculée selon la formule suivante :

$$D = Qd \times \frac{(Cd1out - Cd1in) - (Cd2out - Cd2in)}{Cd2in - Cd1in}$$

où Qd est le débit de liquide de dialyse.

De préférence, selon encore une autre caractéristique de l'invention, le procédé comprend en outre les étapes de :

- déterminer un débit de perfusion Qinf tel que, à la fin de la séance de traitement, la concentration de la substance A dans le milieu intérieur du patient tend vers une concentration souhaitée $[A]_{des}$ ;
- ajuster le débit de perfusion au débit déterminé Qinf.

De préférence, le débit de perfusion Qinf est calculé selon la formule suivante :

$$Q_{inf}= Cl \times \frac{[A]_{des}}{[A]_{sol} - [A]_{des}}$$

où Cl est la clairance du dialyseur pour la substance A.

L'algorithme programmé est exécuté par un appareil de dialyse comprenant :

- des moyens pour préparer un liquide de dialyse contenant du sodium comprenant des moyens de réglage de la concentration en sodium ;
- un circuit de liquide de dialyse comprenant une canalisation d'alimentation et une canalisation d'évacuation, la canalisation d'alimentation ayant une extrémité connectée aux moyens de préparation de liquide de dialyse et une autre extrémité connectable à un dialyseur, la canalisation d'évacuation ayant une extrémité connectable au dialyseur ;
- des moyens pour perfuser à un patient une solution de perfusion contenant du sodium et une substance A à des concentrations déterminées $[Na^+]_{sol}$ et $[A]_{sol}$ ;
- des moyens pour déterminer la concentration en sodium $[Na^+]_{dial}$ du liquide de dialyse pour que le milieu intérieur du patient tende vers une concentration souhaitée en sodium $[Na^+]_{des}$, en fonction de la dialysance D du dialyseur pour le sodium, de la concentration souhaitée en sodium $[Na^+]_{des}$ du milieu intérieur du patient, du débit de perfusion $Q_{inf}$ et de la concentration en sodium $[Na^+]_{sol}$ de la solution de perfusion ;
- des moyens de commande pour piloter les moyens de réglage de la concentration en sodium du liquide de dialyse de façon que cette concentration soit égale à la concentration déterminée $[Na^+]_{dial}$.

D'autres caractéristiques et avantages de l'invention apparaîtront mieux à la lecture de la description qui va suivre. On se reportera à l'unique figure annexée qui représente de façon schématique un système d'hémodialyse selon l'invention.

Le système d'hémodialyse représenté sur la figure comprend un hémodialyseur 1 ayant deux compartiments 2, 3 séparés par une membrane semi-perméable 4. Un premier compartiment 2 a une entrée connectée à une canalisation 5 de prélèvement de sang sur laquelle est disposée une pompe de circulation 6, et une sortie connectée à une canalisation 7 de restitution de sang sur laquelle est interposé un piège à bulles 8.

Un dispositif de perfusion comprenant une pompe 10 et une balance 11 est prévu pour injecter dans le piège à bulles 8 le contenu d'une poche 9 de liquide de perfusion. Le liquide de perfusion est une solution stérile de bicarbonate de sodium dont la concentration en bicarbonate $[HCO3^-]_{sol}$ (c'est-à-dire aussi la concentration en sodium $[Na^+]sol$) est connue. La poche 9 est suspendue à la balance 11 et elle est reliée au piège à bulles 8 par une canalisation 12 sur laquelle est disposée la pompe de perfusion 10. La balance 11 sert à piloter la pompe 10 pour que le débit du liquide de perfusion soit égal à un débit de consigne.

Le second compartiment 3 de l'hémodialyseur 1 a une entrée connectée à une canalisation 12 d'alimentation en liquide de dialyse frais et une sortie connectée à une canalisation 13 d'évacuation de liquide usé (liquide de dialyse et ultrafiltrat).

La canalisation d'alimentation 12 relie l'hémodialyseur 1 à un dispositif de préparation de liquide de dialyse 14 comprenant une canalisation principale 15 dont l'extrémité amont est prévue pour être raccordée à une source d'eau courante. A cette canalisation principale est connectée une canalisation secondaire 16 dont l'extrémité libre est destinée à être immergée dans un conteneur 17 pour une solution saline concentrée contenant du chlorure de sodium, de calcium, de magnésium et de potassium. Une pompe 18 est disposée sur la canalisation secondaire 16 pour permettre le mélange dosé d'eau et de solution concentrée dans la canalisation principale 15. La pompe 18 est pilotée en fonction de la comparaison entre 1) une valeur de consigne de conductivité pour le mélange de liquides se formant à la jonction de la ligne principale 15 et de la ligne secondaire 16 et 2) la valeur de la conductivité de ce mélange mesurée au moyen d'une sonde de conductivité 19 disposée sur la canalisation principale 15 immédiatement en aval de la jonction entre la ligne principale 15 et la ligne secondaire 16.

La canalisation d'alimentation 12 forme le prolongement de la canalisation principale 15 du dispositif 14 de préparation de liquide de dialyse. Sur cette canalisation d'alimentation sont disposés, dans le sens de circulation du liquide, un premier débitmètre 20 et une première pompe de circulation 21.

L'extrémité aval de la canalisation 13 d'évacuation de liquide usé est prévue pour être reliée à l'égout. Sur cette canalisation sont disposés, dans le sens de circulation du liquide, une deuxième pompe de circulation 22 et un deuxième débitmètre 23. Une pompe d'extraction 24 est reliée à la canalisation d'évacuation 13, en amont de la deuxième pompe de circulation 22. La pompe d'extraction 24 est piloté de façon que son débit soit égal à une valeur de consigne de débit d'ultrafiltration dans l'hémodialyseur 1.

La canalisation d'alimentation 12 et la canalisation d'évacuation 13 sont reliées par une première et une seconde canalisations de dérivation 25, 26, qui sont reliées entre elles par une canalisation de jonction 27 sur laquelle est disposée une sonde de conductivité 28. La première canalisation de dérivation 25 est relié à la canalisation d'alimentation 12, en aval de la première pompe de circulation 21, par l'intermédiaire d'une première vanne trois voies 29, et elle est reliée à la canalisation d'évacuation 13, en amont de la deuxième pompe de circulation 22, par l'intermédiaire d'une deuxième vanne trois voies 30. La deuxième canalisation de dérivation 26 et la canalisation de jonction 27 sont reliées par l'intermédiaire d'une troisième vanne trois voies 31.

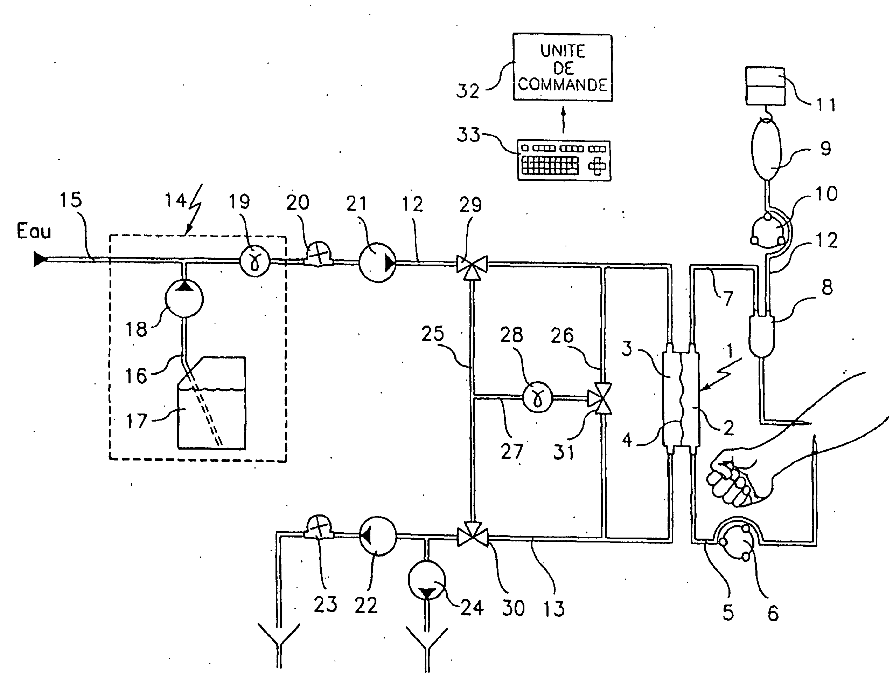

Le système d'hémodialyse représenté sur la figure 1 comprend également une unité de calcul et de commande 32. Cette unité est reliée à une interface utilisateur 33 (clavier alpha-numérique) par laquelle elle reçoit des instructions, telles que diverses valeurs de consigne de débit (débit de sang Qb, débit de liquide de dialyse Qd et, le cas échéant, débit de liquide de perfusion Qinf), de conductivité de liquide de dialyse Cd1 in, Cd2in, de durée de traitement T, de perte de poids WL. Par ailleurs, l'unité de calcul et de commande 32 reçoit des information émises par les organes de mesure du système, tels que les débitmètres 20, 23, les sondes de conductivité 19, 28 et la balance 11. Elle pilote, en fonction des instructions reçues et de modes d'opération et d'algorithmes programmés les organes moteurs du système, tels que les pompes 6, 10, 18, 21, 22, 24 et les vannes 29, 30, 31.

Le système d'hémodialyse qui vient d'être décrit fonctionne de la façon suivante.

Après que le circuit extracorporel de sang a été rincé et rempli de solution saline stérile, il est connecté au patient et, la pompe à sang 6 est mise en service à un débit prédéterminé Qb, 200 ml/min par exemple.

Simultanément, la pompe 18 de dosage de solution concentrée, les pompes de circulation de liquide de dialyse 21, 22 et la pompe d'extraction 24 sont mises en service. Le débit de la pompe de dosage 18 est initialement réglé pour que le liquide de dialyse ait une concentration en sodium (c'est-à-dire aussi une conductivité) telle que la concentration en sodium du milieu intérieur du patient évolue approximativement vers une concentration souhaitée. Le débit Qd de la pompe de circulation 21 disposée sur la canalisation d'alimentation 12 est réglé à une valeur fixe (500 ml/min, par exemple), tandis que le débit de la pompe de circulation 22 disposée sur la canalisation d'évacuation 13 est ajusté en permanence de façon que le débit mesuré par le second débitmètre 23 soit égal au débit mesuré par le premier débitmètre 20. Le débit de la pompe d'extraction 24, qui provoque l'ultrafiltration d'eau plasmatique, est égal au débit Qinf de la pompe de perfusion 10 augmenté du débit de perte de poids (calculé à partir du poids WL qu'il est prescrit de faire perdre au patient et de la durée T de la séance de traitement).

Les vannes 29, 30, 31 sont disposées de façon que le liquide de dialyse frais circule dans la canalisation de jonction 27 et irrigue la sonde de conductivité 28.

La pompe de perfusion 10 est mise en service initialement à un débit Qinf, choisi empiriquement pour que la concentration en bicarbonate du milieu intérieur du patient évolue approximativement vers une concentration souhaitée $[HCO_3^-]_{des}$. Le débit de la pompe 10 est régulé précisément au moyen de la balance 11.

Conformément à l'invention, le débit de la pompe de solution concentrée 18 est ajusté de façon continue pour que la concentration en sodium $[Na^+]_{dial}$ du liquide de dialyse, telle que mesurée au moyen de la sonde de conductivité 19, soit propre à faire évoluer précisément la concentration en sodium du milieu intérieur du patient vers une valeur souhaitée $[Na^+]_{des}$. La concentration en sodium $[Na^+]_{dial}$ du liquide de dialyse est calculée par l'unité de calcul 32 en fonction de la dialysance D du dialyseur 1 pour le sodium, de la concentration souhaitée en sodium $[Na^+]_{des}$ du milieu intérieur du patient, du débit de perfusion Qinf et de la concentration en sodium $[Na^+]sol$ de la solution de perfusion. Elle peut être calculée par application de la formule suivante :

$$[Na^+]dial = \frac{Q_{inf}}{D}\left([Na+]des - [Na+]sol\right) + [Na+]des \qquad (1)$$

La valeur de la dialysance D utilisée dans la formule (1) peut être une valeur fixe estimée empiriquement par l'utilisateur du système de dialyse à partir des performances théoriques du dialyseur utilisé, du débit sang Qb et du débit de liquide de dialyse Qd. Dans ce cas, l'utilisateur fournit cette valeur de la dialysance D à l'unité de calcul et de commande 32 préalablement à la séance de traitement.

De préférence valeur de la dialysance D utilisée dans la formule (1) est une valeur réelle déterminée par le calcul, par exemple par la mise en oeuvre du procédé suivant dont les étapes successives sont commandées par l'unité de commande 32. Les vannes trois voies 29, 30, 31 étant disposées de façon que le liquide de dialyse frais irrigue la sonde de conductivité 28, la conductivité Cd1 in du liquide de dialyse frais correspondant à la prescription est mesurée et est mise en mémoire. Puis les trois vannes 29, 30, 31 sont basculées de façon que la sonde de conductivité 28 soit irriguée par le liquide usé et la conductivité Cd1out de ce liquide est mesurée et mise en mémoire. Le débit de la pompe de concentré 18 est alors modifié (augmenté ou diminué) de façon que la conductivité du liquide de dialyse mis en circulation soit légèrement différente de la conductivité du liquide de dialyse de la prescription. Par exemple, la conductivité du deuxième liquide de dialyse est réglée de façon à être supérieure ou inférieure de 1 mS/cm par rapport à la conductivité du premier liquide de dialyse (laquelle est généralement de l'ordre de 14 mS/cm). Comme précédemment, la conductivité Cd2in du deuxième liquide de dialyse en amont du dialyseur 1 est mesurée et mise en mémoire, après quoi les vannes trois voies 29, 30, 31 sont basculées à nouveau pour que la sonde de conductivité 28 soit irriguée par le liquide usé, et la conductivité Cd2out du liquide usé est mesurée et mise en mémoire. La dialysance D réelle du dialyseur 1 peut alors être calculée par application de la formule suivante :

$$D = Qd \times \frac{(Cd1out - Cd1in) - (Cd2out - Cd2in)}{Cd2in - Cd1in} \qquad (2)$$

Un autre procédé pour calculer la dialysance D réelle à partir de mesures effectuées sur deux liquides de dialyse de conductivités différentes est décrit dans la demande de brevet EP 0 658 352.

Le débit de perfusion Oinf, qui intervient dans le calcul de la concentration en sodium $[Na+]_{dial}$ du liquide de dialyse peut être évalué empiriquement par l'utilisateur du système de dialyse de façon que la concentration en bicarbonate du milieu intérieur du patient évolue approximativement vers une concentration souhaitée. Dans ce cas, l'utilisateur fournit cette valeur de débit de perfusion fixe à l'unité de calcul et de commande 32 préalablement à la séance de traitement.

De préférence, le débit de perfusion Qinf est calculé et est ajusté régulièrement pour que la concentration du sang en bicarbonate tende précisément vers une valeur souhaitée $[HCO3^-]_{des}$. En règle générale, le débit Qinf de la solution de la perfusion peut être calculée à tout moment par application de la formule :

$$Qinf = Cl \times \frac{[HCO3^-]_{des}}{[HCO3^-]_{sol} - [HCO3^-]_{des}} \qquad (3)$$

où Cl est la clairance du dialyseur 1 pour le bicarbonate, qui peut être facilement extrapolée de la dialysance D pour le sodium, quel que soit son mode de détermination.

L'invention ne s'applique pas seulement au mode de dialyse qui vient d'être décrit, où la solution de perfusion comprend du bicarbonate de sodium et où le liquide de dialyse en est dépourvu. Elle s'applique en général à tout mode de dialyse où le liquide de dialyse utilisé contient du sodium et où le patient dialysé reçoit une perfusion d'une solution contenant un sel de sodium.

A titre d'exemple, pour éviter les inconvénients de la dialyse classique au bicarbonate rappelés plus haut, où le liquide de dialyse contient tous les électrolytes du sang et de l'acide acétique, il est possible de mettre en oeuvre le traitement suivant : le liquide de dialyse est préparé à partir d'une solution concentrée de chlorure de sodium et de bicarbonate de sodium, et, éventuellement, de chlorure de potassium (mélange d'eau et de la solution concentrée au moyen de la pompe à débit variable 18 du dispositif représenté sur la figure). Par ailleurs le patient dialysé reçoit une perfusion d'une solution de chlorure de sodium, de magnésium, de calcium et, éventuellement, de potassium. Le potassium figure soit dans la composition du liquide de dialyse, soit dans la composition de la solution de perfusion. La composition de ces liquides est particulièrement adaptée à un traitement par hémodiafiltration où le débit de perfusion est supérieur à un litre par heure.

L'invention qui vient d'être décrite est susceptible de variantes. Au lieu d'une sonde de conductivité unique baignée alternativement par le liquide de dialyse frais et le liquide usé, le circuit de liquide de dialyse peut être équipé de deux sondes de conductivité disposées sur le circuit de liquide de dialyse, respectivement en amont et en aval du dialyseur. Le circuit de liquide de dialyse peut aussi ne comporter qu'une sonde de conductivité disposée en aval du dialyseur, auquel cas les deux valeurs de consigne de conductivité utilisées pour le pilotage de la pompe de concentré 18 pour préparer le premier et le second liquides de dialyse sont substituées dans la formule indiquée ci-dessus, aux valeurs de conductivité mesurées en amont du dialyseur.

**Revendications**

1. Algorithme programmé pour une unité de commande et de contrôle d'un appareil de dialyse, l'appareil de dialyse comprenant :

   - des moyens (14) pour préparer un liquide de dialyse contenant du sodium comprenant des moyens de réglage (18) de la concentration en sodium ;
   - un circuit de liquides de dialyse comprenant une canalisation d'alimentation (12) et une canalisation d'évacuation (13), la canalisation d'alimentation (12) ayant une extrémité connectée aux moyens (14) de préparation de liquide de dialyse et une autre extrémité connectable à un dialyseur (1), la canalisation d'évacuation (13) ayant une extrémité connectable au dialyseur (1);
   - des moyens (9, 10, 12) pour perfuser à un patient une solution de perfusion contenant du sodium et une substance A à des concentrations déterminées [Na+]sol et [A]sol ;
   - une unité de commande et de contrôle dans laquelle l'algorithme programmé, lorsqu'il est exécuté par l'unité de commande et de contrôle dont les moyens pour perfuser comportent une solution de perfusion , rend l'unité de commande et de contrôle capable d'exécuter un procédé de réglage pour que le milieu intérieur du patient tende vers une concentration souhaitée en sodium [Na⁺]des, comportant les étapes suivantes :
   - déterminer la concentration en sodium souhaitée[Na⁺]dial du liquide de dialyse;
   - piloter les moyens (18) de réglage de la concentration en sodium du liquide de dialyse de façon que cette concentration soit égale à la concentration souhaitée déterminée [Na⁺]diall,
   - **caractérisé en ce que** la concentration de sodium souhaitée est déterminée en fonction de la dialysance D du dialyseur (1) pour le sodium, de la concentration souhaitée en sodium [Na⁺]des du milieu intérieur du patient, du débit de perfusion Qinf et de la concentration en sodium [Na⁺]sol de la solution de perfusion.

2. Algorithme programmé selon la revendication 1, **caractérisé en ce que** l'étape de déterminer la concentration en sodium [Na⁺]$_{dial}$ du liquide de dialyse comprend l'étape de calcul cette concentration selon la formule :

$$[Na^+]dial = \frac{Q\ inf}{D}\ ([Na^+]des - [Na^+]sol) + [Na^+]des$$

3. Algorithme programmé selon une des revendications 1 et 2, l'appareil de dialyse comportant une sonde de conductivité (28) pour mesurer la conductivité du liquide de dialyse frais et usé, **caractérisé en ce** l'étape de déterminer la dialysance D du dialyseur (1) pour le sodium comprend les étapes suivantes :

   - préparer et faire circuler successivement dans le dialyseur au moins deux liquides de dialyse ayant des conductivités différentes ;
   - prendre au moins deux mesures de la conductivité d'au moins deux liquides de dialyse en amont et en aval du dialyseur ;
   - calculer la dialysance D à partir des valeurs de conductivité mesurées (Cd1in, Cd1out, Cd2in, Cd2out) dans au moins deux liquides de dialyse préparés successivement.

4. Algorithme programmé selon la revendication 3, **caractérisé en ce que** le calcul de la dialysance D du dialyseur (1) pour le sodium est effectué selon la formule suivante :

$$D = Qd \times \frac{(Cd1out - Cd1in) - (Cd2out - Cd2in)}{Cd2in - Cd1in}$$

où Qd est le débit de liquide de dialyse.

5. Algorithme programmé selon une des revendications 1 à 4, **caractérisé en ce que** le procédé de réglage comprend les étapes suivantes :

- déterminer un débit de perfusion Qinf tel que la concentration de la substance A dans le milieu intérieur du patient tende vers une concentration souhaitée [A]des ;
- piloter les moyens de réglage (10) du débit de perfusion de façon que ce débit soit égal au débit déterminé Qinf.

6. Algorithme programmé selon la revendication 5, **caractérisé en ce que** l'étape de détermination du débit de perfusion Qinf comprend calcul du débit de perfusion Qinf selon la formule :

$$Q_{inf} = Cl \times \frac{[A]_{des}}{[A]_{sol} - [A]_{des}}$$

où Cl est la clairance du dialyseur (1) pour la substance A,

## Patentansprüche

1. Programmierter Algorithmus für eine Steuer- und Überwachungseinheit eines Dialysegeräts, wobei das Dialysegerät:

   - Mittel (14) zur Vorbereitung einer Natrium enthaltenden Dialyseflüssigkeit umfassend Mittel zur Einstellung (18) der Natriumkonzentration;
   - einen Dialyseflüssigkeitskreislauf umfassend einen Zuführungskanal (12) und einen Ablaufkanal (13), wobei der Zuführungskanal (12) ein mit den Mitteln (14) zur Vorbereitung von Dialyseflüssigkeit verbundenes Ende und ein anderes mit einem Dialysator (1) verbindbares Ende aufweist, wobei der Ablaufkanal (13) ein mit dem Dialysator (1) verbindbares Ende aufweist;
   - Mitteln (9, 10, 12) zur Perfusion in einen Patienten von einer Perfusionslösung, die Natrium und einen Stoff A bei vorgegebenen Konzentrationen $[Na^+]_{sol}$ und $[A]_{sol}$ enthält;
   - eine Steuer- und Überwachungseinheit, worin der programmierte Algorithmus - wenn er von der Steuer- und Überwachungseinheit ausgeführt wird - die Steuer- und Überwachungseinheit in der Lage macht, ein Einstellungsverfahren durchzuführen, damit das innere Medium des Patienten zu einer gewünschten Natriumkonzentration $[Na^+]_{des}$ neigt, umfassend die folgenden Schritte:
   - Bestimmung der gewünschten Natriumkonzentration $[Na^+]_{dial}$ der Dialyseflüssigkeit;
   - Steuerung der Mittel (18) zur Einstellung der Natriumkonzentration der Dialyseflüssigkeit, so daß diese Konzentration der bestimmten gewünschten Konzentration $[Na^+]_{diall}$ entspricht;
   - umfasst, **dadurch gekennzeichnet, daß** die gewünschte Natriumkonzentration abhängig von der Dialysance D des Dialysators (1) für Natrium, von der gewünschten Natriumkonzentration $[Na^+]_{des}$ der inneren Medium des Patienten, von der Perfusionsfördermenge $Q_{inf}$ und von der Natriumkonzentration $[Na^+]_{sol}$ der Perfusionslösung bestimmt wird.

2. Programmierter Algorithmus nach Anspruch 1, **dadurch gekennzeichnet, daß** der Schritt von Bestimmung der Natriumkonzentration $[Na^+]_{dial}$ der Dialyseflüssigkeit einen Schritt von Berechnung dieser Konzentration nach der Formel:

$$[Na^+]_{dial} = \frac{Q_{inf}}{D}([Na+]_{des} - [Na+]_{sol}) + [Na+]_{des}$$

umfasst.

3. Programmierter Algorithmus nach einem der Ansprüche 1 und 2, wobei das Dialysegerät einen Leitfähigkeitsfühler (28) zur Messung der Leitfähigkeit der frischen und der gebrauchten Dialyseflüssigkeit, **dadurch gekennzeichnet, daß** der programmierte Algorithmus die Steuer- und Überwachungseinheit in der Lage macht, ein Verfahren zur Bestimmung der Dialysance D des Dialysators (1) für Natrium durchzuführen, umfassend die folgenden Schritte:

- Hintereinandervorbereitung und -zirkulation im Dialysator von mindestens zwei Dialyseflüssigkeiten mit unterschiedlichen Leitfähigkeitswerten;
- Erfassung von mindestens zwei Messungen der Leitfähigkeit von mindestens zwei Dialyseflüssigkeiten aufwärts und abwärts vom Dialysator;
- Berechnung der Dialysance D ausgehend von den gemessenen Leitfähigkeitswerten (Cd1in, Cd1out, Cd2in, Cd2out) in mindestens zwei hintereinandervorbereiteten Dialyseflüssigkeiten.

4. Programmierter Algorithmus nach Anspruch 3, **dadurch gekennzeichnet, daß** die Berechnung der Dialysance D des Dialysators (1) für Natrium nach der folgenden Formel:

$$D = Qd \times \frac{(Cd1out - Cd1in) - (Cd2out - Cd2in)}{Cd2in - Cd1in},$$

worin Qd die Dialyseflüssigkeitsfördermenge darstellt, durchgeführt wird.

5. Programmierter Algorithmus nach einem der Ansprüche 1 bis 4, **dadurch gekennzeichnet, daß** der programmierte Algorithmus die Steuer- und Überwachungseinheit in der Lage macht, ein Einstellungsverfahren umfassend die folgenden Schritte:

- Bestimmung einer Perfusionsfördermenge $Q_{inf}$, so daß die Konzentration des Stoffes A in das inneren Medium des Patienten zu einer gewünschten Konzentration $[A]_{des}$ neigt;
- Steuerung der Mittel zur Einstellung (10) der Perfusionsfördermenge, so daß diese Fördermenge der bestimmten Fördermenge $Q_{inf}$ entspricht,

durchzuführen.

6. Programmierter Algorithmus nach Anspruch 5, **dadurch gekennzeichnet, daß** der Schritt von Bestimmung der Perfusionsfördermenge $Q_{inf}$ die Berechnung der Perfusionsfördermenge $Q_{inf}$ nach der Formel:

$$Q_{inf} = Cl \times \frac{[A]_{des}}{[A]_{sol} - [A]_{des}}$$

umfasst, worin Cl die Clearance des Dialysators (1) für den Stoff A darstellt.

## Claims

1. A programmed algorithm for a control and monitoring unit of a dialysis device, the dialysis device comprising:

- means (14) for preparing a dialysis liquid containing sodium, comprising means for adjusting (18) sodium concentration;
- a dialysis liquid circuit comprising a supply channel (12) and a discharge channel (13), the supply channel (12) having one end connected to the means (14) for preparing the dialysis liquid and another end that can be connected to a dialyzer (1), the discharge channel (13) having one end that can be connected to the dialyzer (1);
- means (9, 10, 12) for perfusing a patient with a perfusion solution, comprising a perfusion solution containing sodium and a substance A at given concentrations $[Na^+]_{sol}$ and $[A]_{sol}$;
- a control and monitoring unit in which the programmed algorithm, when executed by the control and monitoring unit, makes the control and monitoring unit able to execute an adjusting process, so that the patient's internal medium tends towards a desired sodium concentration $[Na^+]_{des}$, comprising the following steps:
- determining the desired sodium concentration $[Na^+]_{dial}$ of the dialysis liquid;
- piloting the means (18) for adjusting the sodium concentration of the dialysis liquid so that said concentration is the same as the determined desired concentration $[Na^+]_{diall}$,
- **characterized in that** the desired sodium concentration is determined as a function of the dialysance D of the dialyzer (1) for sodium, of the desired sodium concentration $[Na^+]_{des}$ of the patient's internal medium, of the perfusion flow rate $Q_{inf}$ and of the sodium concentration $[Na^+]_{sol}$ of the perfusion solution.

2. The programmed algorithm according to claim 1, **characterized in that** the step of determining the sodium concentration $[Na^+]_{dial}$ of the dialysis liquid comprises the step of calculating said concentration according to the following formula:

$$[Na^+]_{dial} = \frac{Q_{inf}}{D}([Na+]_{des} - [Na+]_{sol}) + [Na+]_{des}$$

3. The programmed algorithm according to one of the claims 1 and 2, the dialysis device comprising a conductivity probe (28) for measuring the conductivity of the new and the used dialysis liquid, **characterized in that** the programmed algorithm makes the control and monitoring unit able to execute a process for determining the dialysance D of the dialyzer (1) for sodium, comprising the following steps:

   - preparing and circulating sequentially in the dialyzer at least two dialysis liquids having different conductivities;
   - taking at least two conductivity measures of at least two dialysis liquids upstream and downstream from the dialyzer;
   - calculating the dialysance D starting from the conductivity values as measured (Cd1in, Cd1out, Cd2in, Cd2out) in at least two dialysis liquids prepared sequentially.

4. The programmed algorithm according to claim 3, **characterized in that** the dialysance D of the dialyzer (1) for sodium is calculated according to the following formula:

$$D = Qd \times \frac{(Cd1out - Cd1in) - (Cd2out - Cd2in)}{Cd2in - Cd1in}$$

where Qd is the dialysis liquid flow rate.

5. The programmed algorithm according to one of the claims 1 to 4, **characterized in that** the programmed algorithm makes the control and monitoring unit able to execute an adjusting process comprising the following steps:

   - determining a perfusion flow rate $Q_{inf}$ so that the concentration of substance A in the patient's internal medium tends towards a desired concentration $[A]_{des}$;
   - piloting the means for adjusting (10) the perfusion flow rate so that said flow rate is the same as the determined flow rate $Q_{inf}$.

6. The programmed algorithm according to claim 5, **characterized in that** the step of determining the perfusion flow rate $Q_{inf}$ comprises the calculation of the perfusion flow rate $Q_{inf}$ according to the following formula:

$$Q_{inf} = Cl \times \frac{[A]_{des}}{[A]_{sol} - [A]_{des}}$$

where Cl is the clearance of the dialyzer (1) for substance A.

UNITE
DE
COMMANDE

Eau